(19) Europäisches Patentamt
European Patent Office
Office européen des brevets

(11) Numéro de publication: **0 356 355 A1**

(12) **DEMANDE DE BREVET EUROPEEN**

(21) Numéro de dépôt: **89420308.2**

(22) Date de dépôt: **22.08.89**

(51) Int. Cl.$^5$: **A 61 K 9/06**
A 61 K 47/32, A 61 F 9/00

(30) Priorité: **22.08.88 US 234755**

(43) Date de publication de la demande:
**28.02.90 Bulletin 90/09**

(84) Etats contractants désignés:
**AT BE CH DE ES FR GB GR IT LI LU NL SE**

(71) Demandeur: **Baron, Neville A.**
**146 Sandpiper Key**
**Secaucus New Jersey 07094 (US)**

(72) Inventeur: **Baron, Neville A.**
**146 Sandpiper Key**
**Secaucus New Jersey 07094 (US)**

(74) Mandataire: **Dupuis, François**
**Cabinet Charras 3 Place de l'Hôtel-de-Ville BP 203**
**F-42005 St. Etienne Cédex 1 (FR)**

(54) **Liquide ophtalmique pour lunettes de soleil.**

(57) Les dosages ophtalmiques de chromophores dans une gelée acqueuse afin de bloquer les transmissions de tous les spectres, ou de spectres variés d'U.V, vers les yeux, lorsqu'ils sont utilisés en tant que liquide ophtalmique pour lunettes de soleil.

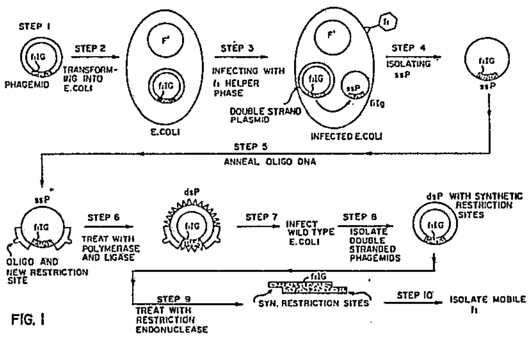

FIG. 1

EP 0 356 355 A1

**Description**

## Liquide ophtalmique pour lunettes de soleil.

### Antécédents de l'invention

Ceci est en partie la continuation de la demande faite aux U.S, numéro de série 935,843, enregistrée le 28 Novembre 1986, et composée comme suit:

(1) Chromophores additionnels de dérivés ethyléniquement non-saturés de dihydroxy benzophénones en tant que liquide pour lunettes de soleil;

(2) Le pourcentage de transmission d'une longueur d'ondes particulière de lumière utilisant certaine quantité de liquide pour lunettes de soleil;

(3) Une méthode d'injection ou de tatouage des chromophores sous l'épithélium cornéen, afin d'établir une haute absorption ou une basse transmission des rayons de lumière nocifs.

Bien que les rayons ultra-violets aient longtemps été reconnus comme un facteur de développement du cancer de la peau, de vieillissement de la peau, et de changements mutagéniques, c'est seulement pendant les dix dernières années ou moins, que les rayons ultra-violets ont été universellement reconnus comme un facteur causatif dans la pathogénèse occulaire.

Chez les humains, l'oeil s'est transformé en un organe sophistiqué ayant des réactions neurophysiologiques aux photons dans une certaine portion du spectre électromagnétique, qui fournit une carte constamment détaillée de l'environnement immédiat. L'action du spectre à ces réactions réside principalement à l'intérieur du cercle de longueur d'ondes de 400-700nm, qui a été appelé le spectre visible ou "lumière".

Le maximum des réactions du spectre de l'oeil correspond approximativement au maximum d'irradiation spectrale solaire. A cause de la présence de rayons solaires UV pendant la plupart des heures du jour, l'oeil peut être exposé quotidiennement à une certaine quantité de rayons ultra-violets solaires tout au long de la vie. Les longueurs d'ondes plus courtes qu'environ 290nm ou UV-C sont partiellement ou complètement absorbées à l'intérieur de la cornée et de la membrane conjonctive. Les effets aigus de l'exposition à ces longueurs d'ondes sont essentiellement ceux de la conjonctivite et de l'inflammation de la cornée connus sous le nom de photokeratite. La réaction inflammatoire de la couche la plus extérieure de l'oeil aux rayons UV-C et UV-B peut être similaire à celle de la peau à certains égards.

Le progrès clinique ou la description de la photokeratite suit un cours caractéristique. Par exemple, après exposition, il y a une période d'attente qui varie quelque peu inversement avec la quantité d'exposition. La période d'attente peut être aussi courte que 30 mn ou aussi longue que 24 heures, mais elle est en général de 6 à 12 heures.

La conjonctivite, qui est souvent accompagnée par un érythème de la peau entourant les paupières, est associée à la sensation d'un corps étranger ou de "sable" dans les yeux, avec des degrés variables de photophobie (intolérance à la lumière), sécrétion excessive de larmes (pleurs), et de blépharospasmes (spasmes des muscles de la paupière). La douleur cornéenne peut être très forte, et l'individu est dans l'incapacité de faire quoi que ce soit pendant un certain moment. Ces symptômes aigus durent habituellement de 6 à 24 heures, et presque tout l'inconfort disparait au bout de 48 heures. La conjonctivite résultant de l'exposition occasionne très rarement des dommages pemanents.

Toutefois, contrairement à la peau, le système occulaire ne développe pas de tolérance à l'exposition répétée des ultra-violets. Les diminutions ou les augmentations de groupes de cellules épithéliales cornéennes conduisent à un pointillé visiblement reconnaissable ou à une mosaique irrégulière granulée de la surface cornéenne. Avec des doses d'UV plus importantes que le seuil requis pour la photokeratite, les cellules de la surface épithéliale montrent une fragmentation nucléaire, les cellules du milieu de la surface épithéliale montrent une formation de vacuoles, et les cellules basales montrent une inhibition de mitosis et un stroma cornéen trouble apparait. L'inflammation est aussi présente dans la membrane conjonctive où la vasodilatation, l'oedème, et les cellules inflammatoires, infiltrées dans celle-ci, sont suivis de desquamation.

Parce que les longueurs d'ondes plus longues que 290nm sont largement transmises par la cornée, le cristallin de base et l'iris sont exposés aux UV-A. Le cristallin absorbe essentiellement tous les UV-A qui le frappent, et est par conséquent, le tissu occulaire particulièrement susceptible d'altération par l'exposition de l'oeil aux UV-A. La production possible de cataracte lenticulaire chez les humains par l'exposition aux UV-A est par conséquent une cause d'inquiétude majeure.

Toutes les altérations du cristallin ou de sa capsule qui résultent d'une transmission apparement affaiblie ou de diffusion amoindrie de la lumière visible peuvent être appellées cataracte. Des altérations minimes, bien que détectables par un examen microscopique minutieux, ne causent aucun changement dans l'acuité visuelle habituelle, mais des altérations plus marquées de transmission de la lumière peuvent déteriorer ou éliminer la vision. Le terme de cataracte est souvent réservé à ces diminutions symptômatiques de la vision.

## Récapitulation de l'invention

En conséquence, c'est l'objectif principal de la présente invention de fournir une dispersion acqueuse contenant un chromophore occulaire qui établisse un équilibre du spectre d'absorption des ultra-violets, entre la pellicule de larmes et l'épithélium de la cornée des mammifères, dans les longueurs d'ondes UV-A et UV-B.

Un autre objectif de la présente invention est de fournir une dispersion acqueuse contenant un chromophore occulaire qui établisse un équilibre du spectre d'absorption des ultra-violets, entre la pellicule de larmes et l'épithélium de la cornée des mammifères, qui ne soit pas toxique pour l'épithélium de la cornée dans les concentrations fonctionnelles.

Un objectif supplémentaire de la présente invention est de fournir une dispersion acqueuse contenant un chromophore occulaire en même temps qu'un polymère véhicule chargé en molécules lourdes qui maintienne l'équilibre du spectre d'absorption des ultra-violets entre la pellicule de larmes et l'épithélium de la cornée des mammifères pendant environ 2 à 4 heures.

En général, l'objectif de l'invention est réalisé en préparant une dispersion acqueuse contenant environ de 1 à 10% par poids de graisse ou de lipide soluble chromophore de 2 éthylexyl par méthoxycinnamate, le même pourcentage d'un mélange de 2 éthylexyl-p-méthoxycinnamate et d'octyl méthoxycinnamate avec un polymère chargé en molécules lourdes, ou des dérivatifs éthyléniquement non-saturés de dihydroxy benzophénones sélectionnés à partir du groupe consistant en:

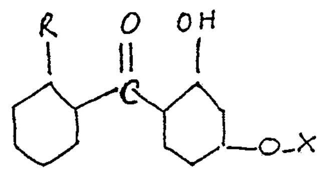

ET

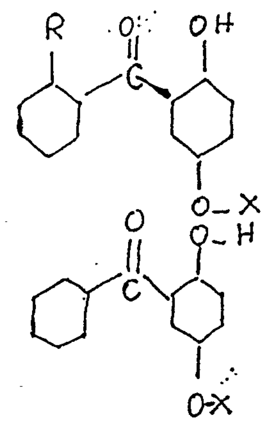

ET

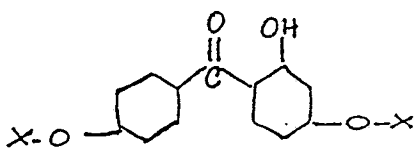

ET

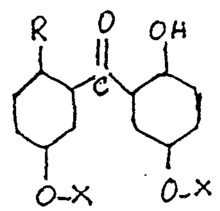

où X est un radical éthyléniquement non-saturé, sélectionné à partir du groupe composé de acryloxybetahydroxypropyl et où dans R, il y a un radical sélectionné à partir du groupe composé d'hydrogène et d'hydroxyl, et où il s'agit d'appliquer des gouttes de ladite dispersion directement dans l'oeil d'un sujet mammifère.

Ceci, aisi que d'autres buts de l'invention deviendront plus apparents avec la description détaillée suivante.

## Description détaillée de l'invention

Tandis que le spectre visible de la lumière est d'environ 400nm et au-dessus, les Ultra-Violets A, ou UV-A sont d'à peu prés 400nm à 325nm. La portée des Ultra-Violets B ou UV-B est d'environ 325nm à environ 290nm.

L'absorption des rayons par la cornée et le cristallin de l'oeil humain est telle que très peu de radiation de longueur d'onde plus courte que 390nm, ce qui rentre dans la portée des UV-A, atteint la rétine. Pour les individus sans cristallin, beaucoup d'UV-A frappant l'oeil, atteignent la rétine. L'irradiation d'UV-A d'animaux normaux avec et sans la présence de composants UV-A sensibilisants, produit des dommages à la rétine.

De multiples cobayes animaux on été utilisés pour étudier les altérations biochemiques dans la rétine par les UV-A. Par exemple, les études d'animaux nécrophages dénués de tout radical, indiquent apparement que les UV-A déteriorent la rétine des dindes par une péroxydation des lipides, et les protéines et les synthèses de RNA dans les batonnets rétiniens des requins ont été supprimés par irradiation à 340-380nm. Cependant,

dans les extraits de protéine, la synthèse de protéine de la rétine du rat était supprimée par des radiations de 320nm et non par des expositions de 340 ou 360nm.

Les UV-A produits par des changements morphologiques ou histologiques dans la rétine ont été étudiés en exposant l'oeil intact entier de souris. Dans cette étude, on a noté qu'après 10 semaines d'exposition à des radiations de 365nm, le photorécepteur extérieur aux segments rétiniens de la souris devenait plus mince. Les animaux ont été exposés pendant 12 heures chaque jour aux UV-A de lampes fluorescentes (450uW/cm2). La seizième semaine, les batonnets extérieurs aux segments étaient encore plus détruits et les débris restants étaient partiellement digérés par les cellules phagocytes.

Le rayon laser aux alentours de 350-365nm cause également des dommages aux segments extérieurs de la rétine des singes rhésus, et l'exposition systématique des rats aux UV-A à haute intensité pendant plus de trois ans, conduit à une atrophie des premiers neurones, une dégénération partielle des deuxièmes neurones, et une destruction de la structure rétinienne.

Donc, il y a des raisons de croire que les UV-A provoquent photochimiquement des lésions rétiniennes, si la transmission spectrale occulaire des espèces en questions permet aux UV-A d'atteindre la rétine.

De plus, les expositions expérimentales d'animaux et d'humains indiquent que pour des longueurs d'ondes plus courtes que 310nm environ, ce qui fait partie des UV-B ou, 325-290nm de longueur d'ondes, la kératite et l'altération de la cornée sont les risques occulaires majeurs. Ces réactions et ces effets sont habituellement douloureux mais réversibles. Cette altération de la cornée provoquée par les UV semble suivre un mécanisme photochimique.

Les longueurs d'ondes Ultra-violet plus longues que 290nm peuvent atteindre le cristallin, l'iris, et l'humeur acqueuse (la chambre antérieure). La preuve expérimentale sur les lapins et les singes montre que les cataractes lenticulaires permanentes peuvent être produites par une seule haute irradiation ou une très longue durée d'exposition aux UV-A ou aux UV-B. De plus, chez les souris albinos, les cataractes peuvent être produites par des expositions multiples et quotidiennes aux UV-A, qui sont au dessous du simple seuil de dose d'exposition des dommages cornéens observables.

Les études biochemiques sur les cristallins humains et sur ceux d'autres mammifères montrent que les UV-A semblent provoquer des cataractes lenticulaires par le biais d'une altération des protéines du cristallin, dont les molécules d'un poids très faible à l'état soluble, deviennent plus lourdes, plus denses, après le changement à l'état insoluble, ce qui peut causer une dispersion de la lumière à l'intérieur du cristallin (la cataracte), et il y a des preuves que l'exposition quotidienne à long terme aux UV-A peut détruire la rétine des souris.

G.Klecak[1] a dirigé des études sur l'utilisation de 2-ethylhexyl-p-méthoxycinnamate seul ou avec de l'octyl méthoxycinnamate, pour déterminer les réactions de ces composants qui protègent de la lumière, dans l'étude de l'influence des UV-A et UV-B.

(1. " Determination of the Lightscreening Activity in the UV-A Range of 8 Preparations in the Animal Model". GIVAUDAN S.A, avril 1985. "Determination of the Lightscreening Activity in the UV-A Range of 6 Preparations in the Animal Model". GIVAUDAN S.A, Avril 1985. "Determination of the Lightscreening Activity in the UV-B Range" GIVAUDAN S.A, Avril 1985.)

La caractérisation de l'activité protectrice du soleil de ces composants sur les humains dans l'étude de l'influence des UV-A, a également été dirigée par G. Klecak et d'autres[2]; cependant, le potentiel de ces composants n'a pas été déterminé dans le but d'une utilisation en tant que liquide pour lunettes de soleil actuellement appliqué aux yeux.

(2. "Determination of the Light Protective Activity in the UV-A Range of 2 Sunscreen Preparations On Patients Under PUVA-TREATMENT". GIVAUDAN S.A, Avril 1985.)

La présente invention est axée sur une dispersion acqueuse d'un chromophore et d'un polymère chargé en molécules lourdes qui forme une dispersion visqueuse, et est utilisée pour établir un équilibre du spectre d'absorption des UV-A et UV-B entre la pellicule de larmes et l'épithélium de la cornée des mammifères, lorsqu'elle est appliquée aux yeux.

La prolongation de l'équilibre semble être réalisée par la lente libération du chromophore de la solution visqueuse, et/ou par la lentre érosion de la surface visqueuse. Le chromophore contenant les compositions visqueuses ou gelée de la présente invention, ont un temps de rétention prolongé dans les yeux, et restent an contact avec la surface de l'oeil pendant des périodes d'environ 2 à 4 heures.

La présente invention est dirigée vers une dispersion acqueuse d'un chromophore et d'un polymère chargé en molécules lourdes qui forme une dispersion visqueuse et peut être utilisée pour prolonger la durée du chromophore lorsque la gelée est appliquée dans l'oeil. La prolongation de l'activité du chromophore est réalisée à travers une lente libération de la matrice de la gelée, et/ou à travers une lente érosion de la surface de la gelée. Aussi, le chromophore contenant la composition de la gelée de la présente invention a un temps de rétention prolongé dans l'oeil, et reste en contact avec la surface de l'oeil pour une période de temps étendue.

Les polymères utilisés dans la présente invention ont un poids moléculaire d'environ 1 million à environ 6 millions, et sont caractérisés par des groupes fonctionnels carboxyliques et anhydrides, et contiennent de préférence entre 2 et 7 atomes de carbone par groupe fonctionnel. la gelée qui se forme durant la préparation de la dispersion chromophore/polymère, a une viscosité d'environ 40,000 à environ 300,000 cps à 20 rpm (axe 7), à 25 degrés centigrades, généré par un Viscomètre Brookfield RVT, et de préférence d'environ 75,000 à environ 200,000 cps. Les polymères utiles et adéquats pour la présente invention sont le carboxypolyméthylène, un polymère de carboxy vinyle, (disponible sous le nom commercial de Carbopol, de la Société B.F. Goodrich); et de l'éthylène maléique anhydride (disponible sous le nom commercial de EMA de la Société Monsanto). Les polymères utilisés dans la composition de la gelée sont d'une quantité d'environ 2 à 8 pour cent par poids.

La substance du chromophore est présente dans la composition de la gelée en quantité suffisante pour former effectivement une pellicule dans l'oeil entier, et établir ainsi un équilibre du spectre d'absorption des ultra-violets entre la pellicule de larmes et l'épithélium de la cornée, contre les longueurs d'ondes UV-A et UV-B. Les quantités non-toxiques des composants du chromophore qui réalisent cet effet sont d'environ 1 à 8% par poids.

La cornée est vue comme une sorte de sandwich graisse-eau-graisse. Les analyses chimiques montrent que le contenu en lipides de l'épithélium et de l'endothélium est 100 fois plus grand que celui du stroma cornéen. Il en résulte que l'épithélium et l'endothélium sont relativement imperméables aux électrolytes, mais sont aisément pénétrés par les substances solubles dans la graisse.

La proportion des longueurs d'ondes en nonomètres (NM) des UV-C, UV-B, UV-A et de la portée visuelle utilisant des solutions préparées selon les exemples 1, 2 et 3, et contenant de l'octyl méthoxycinnamate (CMC), 4-t-butyl-4-méthoxy-dibenzoylméthane (BMDM), un mélange de 50/50 pour cent de OMC et de BMDM et 2-hydroxy-4(2-hydroxy-3-méthacrylyloxy) propoxybenzophénone (HHMPB), en tant que liquide pour lunettes de soleil, mis goutte à goutte dans l'oeil d'un homme de 22 ans pour obtenir un blocage total ou 0 (zéro) transmission d'U.V, est défini comme suit:

| longueur d'ondes (NM) | Cristallin humain (22 ans) | Liquide lunettes soleil OMC | Liquide lunettes soleil BMDM | OMC + mélange liquide lunettes de soleil | Liquide lunettes soleil HHMPB |
|---|---|---|---|---|---|

UV-C

| | | | | | |
|---|---|---|---|---|---|
| 230 | 0 % | 45 % | 40 % | 23 % | 0 % |
| 250 | 0 % | 32 % | 38 % | 10 % | 0 % |
| 270 | 0 % | 20 % | 34 % | 3,3% | 0 % |

UV-B

| | | | | | |
|---|---|---|---|---|---|
| 290 | 0 % | 4 % | 30 % | 3 % | 0 % |
| 310 | 0,6 % | 2 % | 25 % | 0,7 % | 0,3 % |

UV-A

| | | | | | |
|---|---|---|---|---|---|
| 320 | 0,7 % | 4 % | 10 % | 0,5 % | 0,3 % |
| 330 | 0,8 % | 8 % | 5 % | 0,5 % | 0,4 % |
| 340 | 0,9 % | 36 % | 1 % | 0,5 % | 0,5 % |
| 350 | 0,9 % | 72 % | 0,7 % | 0,5 % | 0,6 % |
| 360 | 0,9 % | 95 % | 0,7 % | 3 % | 0,9 % |
| 370 | 0,9 % | 0 % | 1 % | 3,5 % | 1,1 % |
| 380 | 0,9 % | 0 % | 5 % | 5,5 % | 1,2 % |
| 390 | 5,0 % | 0 % | 13 % | 14,5 % | 1,5 % |
| 400 | 10,0 % | 0 % | 29 % | 28,5 % | 6,0 % |

Visuel

| | | | | | |
|---|---|---|---|---|---|
| 410 | 15,0 % | 100 % | 50 % | 50 % | 18,0 % |
| 420 | ' 81 % | 100 % | 70 % | 70 % | 38,0 % |
| 430 | 83 % | 100 % | 88 % | 88 % | 74,0 % |
| 440 | 84 % | 100 % | 95 % | 94 % | 80,0 % |
| 450 | 85 % | 100 % | 100 % | 100 % | 88,0 % |

OMC + BMDM = préparation 50% + 50%
*Un blocage total est considéré comme 0 (zéro) transmissions d'UV à 400 NM.
A) UV-A BMDM 1.5-4.0% (blocage total UV-A).
B) UV-B OMC 2.0-7.5% (blocage total UV-B).
C) Combinaison de OMC + BMDM 1.0-8.0% Blocage total UV.
D) HHMPB 1.0-8.0% = Blocage total UV-B et blocage partiel UV-A @ 5.5%.
** E) A l'age de 55 ans, le cristallin normal humain est affecté par des changements prématurés concernant la cataracte, donc, UV-A et V.L ne sont pas transmis dans la même proportion qu'à 22 ans.
***Le polymère éthylène-maléique anhydride, charge en molécules lourdes formant la gelée, sans le chromophore a une proportion d'environ 88% à 92% à partir d'une longueur d'ondes de 320nm à environ 420nm.

2-éthylhexyl-p-méthoxycinnamate octyl méthoxycinnamate, et des dérivés éthyléniquement non-saturés de dihydroxybenzophénones sont les chromophores qui sont solubles dans la graisse, et la dispersion de ces chromophores dans le polymère chargé en molécules lourdes pour être placés directement dans l'oeil, peut être réalisée par n'importe quel moyen adéquat.

Plusieurs méthodes sont utilisées pour préparer les compositions polymère/chromophore de l'invention. La première méthode, I, consiste à disperser le polymère dans l'eau, suivi par l'addition d'un chromophore basique pour neutraliser le polymère. La neutralisation est responsable de la formation d'un complexe hydrogel du chromophore et du polymère. Le ph final dépend du taux basique du chromophore et de la quantité ajoutée. Si le chromophore n'est pas suffisament basique, le ph de l'hydrogel est ajusté en ajoutant une substance basique telle que l'ammonium hydroxyde, le sodium hydroxyde, l'éthanolamine, ou tout autre composant basique pour fournir le ph désireé. Le ph idéal est d'environ 4,5 à 8,5 dans les formulations de la gelée polymère/chromophore. On devrait noter également que le chromophore peut être ajouté à un hydrogel formé par l'addition d'un agent basique au polymère pour former d'abord la gelée, suivi de l'ajout du chromophore dans la concentration désirée.

Dans une deuxième méthode, II, un sel du chromophore et du polymère est préparé. Le sel chromophore est préparé en dispersant le polymère dans un solvant organique inerte, tel que l'hexane, le benzène ou le chloroforme, afin de former une sub stance liquide très mince. Par la suite, une solution du chromophore dans le solvant est ajoutée à cette substance. Une réaction de neutralisation de base-acide se produit dans ce que le résultat du polymère chromophore précipite à partir du solvant. Après la suppression du solvant, il reste une poudre solide. La masse solide peut être réduite à de fines particules en l'écrasant. Par la suite, une gelée est préparée par la dispersion de la poudre finement divisée, dans de l'eau.

Une troisième méthode, III, utilise la forme de sel acide du chromophore. Un base, telle que le sodium hydroxyde, est utilisée pour neutraliser une dispersion acqueuse du polymère, et forme une gelée suivie de l'addition de la forme de sel acide du chromophore.

La durée de l'activité des formulations de la gelée contenant les chromophores préparés par ces trois méthodes est substantielle. Par exemple, 2-éthylexyl-p-méthoxycinnamate avec un carboxypolyméthylène par la méthode I donnent des formulations de gelée qui restent dans la poche conjonctivale d'un lapin pendant 2 à 4 heures. Afin de formuler de façon plus détaillée l'invention, les très petites particules de sel chromophore du polymère préparé selon la méthode II, sont suspendues dans un véhicule non-acqueux, tel que l'huile stabilisée, e.g. l'huile minérale, l'huile végétale et du fluide silicone. Par la suite, les particules suspendues sont administrées directement dans l'oeil. Une gelée se forme entre la pellicule de larmes et l'épithélium de la cornée. Les sels 2-ethylhexyl-p- méthoxycinnamate de carboxypolyméthylène, contenant 4 à 6% par poids par dose, sont actifs en tant qu'absorbeurs d'U.V sur les yeux d'un lapin albinos pendant 2 à 4 heures.

Les exemples suivants illustreront davantage les qualités diverses de l'invention, sans limiter les possibilités de l'invention, qui sont mises en avant dans les affirmations suivantes:

Exemple I:

Un type de sel de carboxypolyméthylène et 2-ethylhéxyl-p-méthoxycinnamate est préparé selon la méthode II. Six grammes de carboxypolyméthylène (disponible sous le nom comercial de Carbopol 940 de la Société B.F.Goodrich) sont remués dans 30 ml d'hexane. Quatre grammes de 2-éthylhéxyl-p-méthoxycinnamate sont dissouts dans 30 ml d'hexane. La solution 2-éthylhéxyl-p-méthoxycinnamate est alors mélangée avec la suspension de carboxypolyméthylène. Une réaction de neutralisation acide-base se produit pour former un sel de 2-éthylhéxyl-p-méthoxycinnamate et du polymère. Le type de sel du polymère et de 2-éthylhéxyl-p-méthoxycinnamate est récupéré à partir du porteur d'hexane sous la forme d'une poudre très fine contenant environ 35 à 40 pour cent par poids de 2-éthylhéxyl-p-méthoxycinnamate.

Deux formulations de gelée sont préparées, contenant le sel 2-éthylhéxyl-p-méthoxycinnamate/carboxypolyméthylène. Les formulations de la gelée contiennent les éléments indiqués ci-dessous dans le tableau 1 à la quantité indiquée.

Tableau I.

| Ingrédients | Formulation A | % par poids formulation B |
|---|---|---|
| sel 2-éthylhéxyl-p-méthoxycinnamate/Carboxypolyméthylène. | 6.0 | 6.0 |
| Benzalkonium Chloride (U.S.P.) | 0.01 | 0.01 |
| Sodium hydroxyde (3N) approx. | ph 5.35 | approx 5.35 |
| Eau purifiée | 94 | 94 |

Obtenir 100 grs de gelée complètement terminée consiste en l'ajout de Benzalkomium Chloride à 80 grs d'eau purifiée. La solution de Benzalkonium Chloride est remuée en même temps qu'on ajoute le type de sel sous forme de poudre de carboxypolyméthylène/2-éthylhéxyl-p-méthoxycinnamate. La solution est remuée pendant une ou deux minutes, pour mouiller la poudre de sel autant que possible avant la formation de la gelée. On continue de remuer jusqu'à ce qu'il n'y ait plus d'autre hydratation apparente. On ajoute alors le sodium hydroxyde en augmentant petit à petit pour arriver au ph indiqué. L'eau purifiée est alors ajoutée à la gelée pour l'alourdir jusqu'à 100grs. La préparation de la gelée est autoclavée à 120 degrés centrigrades, pendant 20 minutes, suivi par une rapide évacuation. Toute bulle d'air dans la gelée ainsi obtenue peut être retirée par centrifugation.

Si l'on utilise le Viscomètre Ferranti-Shirley sous les conditions suivantes: 3X Switch position, 60 tours par seconde,un cône moyen et avec une tension constante de 100 rpm, on obtient les degrés de viscosité suivants:

Tableau II.

| | A 25*C | A 37*C | B 25*C | B 37*C |
|---|---|---|---|---|
| Plastique Viscosité (CPS) | 740 | 708 | 546 | 804 |

Appliquer les compositions de la gelée chromophore, placées dans l'oeil, au goutte à goutte, fournit une pellicule, équilibre du spectre d'absorption des U.V entre la pellicule de larmes et l'épithélium de la cornée; et, en plus d'absorber la lumière des ultra-violets que des lunettes de soleil normales auraient absorbée, la gelée absorbe environ 30 à 35% des radiations UV-A et UV-B qui atteignent normalement l'oeil par-dessus, par-dessous et sur les côtés de lunettes de soleil normales.

De plus, les compositions de la gelée de l'invention contiennent environ 92 pour cent par poids d'eau dans la matrice du polymère, et de ce fait, sont claires. En conséquence, la vision du sujet traité avec la gelée ne devient par troublés car l'index réfractaire de la gelée est similaire à celui des larmes.

Exemple 2

En accord avec la méthode III, la forme de sel acide du mélange 50/50 de 2-éthylhéxyl-p-méthoxycinnamate/ octyl-méthoxycinnamate est incorporée l'intérieur d'une gelée faite de éthylène maléique anhydride (disponible sous le nom commercial de EMA-91 de Monsanto). Deux préparations de gelée sont préparées et contiennent les éléments indiqués dans le tableau III ci-dessous.

Tableau III.

| Eléments | % par poids<br>Formulation A | <br>Formulation B |
|---|---|---|
| Ethylène maléique anhydride | 3.38 | 5.0 |
| 2-éthylhéxyl-p-méthoxycinnamate hydrochloride/ ocyl mthoxycinnamate hydrochloride | 4.0 | 4.0 |
| 28% ammonium hydroxyde | 2.27 | 3.5 |
| Mannitol, N.F. | 1.0 | 2.0 |
| Benzalkonium chloride (U.S.P.) | 0.01 | 0.01 |
| Eau purifiée (Balance à 100%) | Balance | |

Pour préparer 100 grs de gelée terminée, il faut ajouter l'éthylène maléique anhydride au vortex de 25 ml d'eau vigoureusement remuée en utilisant un mélangeur à grande vitesse. Une minute de mélange est suffisante pour mouiller et disperser le polymère. L'ammonium hydroxyde a été ajouté à la dispersion et mélangée pendant une ou deux minutes jusqu'à ce qu'une gelée solide soit formée. 2-éthylhéxyl-p-méthoxycinnamate, hydrochloride/octyl méthoxycinnamate hydrochloride, mannitol et benzalkronium chloride sont dissous dans 15 ml d'eau purifiée et ajoutés à la gelée. Ce mélange est remué pendant 4 minutes et l'on obtient un ph lisible d'approximativement 5.l.

En utilisant un Viscomètre Brookfield RVT à 20 rpm équipé d'un axe 7, et utilisant également un Viscomètre Ferranti-Shirley sous les conditions suivantes: 3X Switch position, 60 tours par seconde, avec un cône moyen et 100 rpm constants, les déterminations de viscosité suivantes sont obtenues:

| | 24°C | 25°C | 37°C |
|---|---|---|---|
| | | A | |
| Viscosité brookfield (cps) | 123,000 | - | - |
| Viscosité plastique Ferranti-Shirley (cps) | | 434 | 384 |
| | | B | |
| Viscosité Brookfield (cps) | 109,000 | - | - |
| Viscosité plastique Ferranti-Shirley (cps) | - | 692 | 558 |

Parmi les moyens adéquats pour placer les chromophores de l'invention dans l'oeil, il y a le "tatouage", ou technique d'injection pour administrer le chromophore sous l'épithélium cornéen et dans le stroma à un point proche de la membrane de Descemet et de l'endothélium, afin de fournir par l'intermédiaire d'un implant, une barrière permanente à semi-permanente aux U.V, qui serait éventuellement dissipée par le métabolisme normal des tissus, ou par une dissipation physique. L'injection, ou le tatouage, est réalisé en utilisant un mécanisme d'injection d'un jet hypodermique qui fonctionne hydrauliquement, pour injecter le chromophore jusqu'à une épaisseur d'environ 2 à 3 microns. Le mécanisme injecte rapidement des quantités mesurées de chromophore à une haute pression et à grande vitesse à travers la pointe d'un jet. L'agent chromophore opère sa propre ouverture dans le stroma, et pénètre jusqu'à une épaisseur adéquate pour l'implantation. On effectue les ajustements du dosage d'environ 0.1 à 1.0 cc en tournant un instrument de réglage situé à l'arrière du mécanisme. Chaque tour de 360 degrés de l'instrument de réglage, est égal à 0.1 cc.

L'on peut effectuer beaucoup de changements avec l'utilisation de la gelée-chromophore de l'invention, et l'on peut envisager que le chromophore per se peut être placé dans un cristallin intra-occulaire avant la pose d'une lentille dans l'oeil, afin de faire écran aux rayons UV-A et UV-B.

**Revendications**

1). Un dosage ophtalmique d'une geléee acqueuse pour établir un blocage total de la transmission des UV-B, lorsqu'elle est placée dans les yeux, ladite gelée comprenant un chromophore d'octyl méthoxycinnamate, et un polymère éthylène-maléique anhydride chargé en molécules lourdes formant une gelée, ayant un poids moléculaire d'au plus 1.000.000; ledit chromophore étant présent à une quantité d'environ 2 à 7.5% pour cent par poids, ledit polymère est présent à une quantité d'environ 2 à 8 pour cent par poids, et ladite gelée a une viscosité d'environ 40.000 à 300.000 cps.

2). Un dosage ophtalmique d'une gelée acqueuse pour établir un blocage total des transmissions d'UV-B et un blocage partiel des UV-A lorsqu'il est placé dans les yeux, ladite gelée comprenant un chromophore de 2-hydroxy-4-(2-hydroxy-3-métacrylyloxy) propoxybenzophénone, et un polymère éthylène-maléique anhydride, chargé en molécules lourdes formant une gelée, ayant un poids moléculaire d'au plus 1.000.000; ledit chromophore étant présent à une quantité d'environ 1 à 8 pour cent par poids, ledit polymère est présent à une quantité d'environ 2 à 8 pour cent par poids, et ladite gelée a une viscosité d'environ 40.000 à 300.000 cps.

3). Un dosage ophtalmique d'une gelée acqueuse pour établir un blocage total de la transmission des UV-A à environ 400nm lorsqu'elle est placée dans les yeux, ladite gelée comprenant un chromophore de 4-t-butyl-4-méthoxy-dibenzoylméthane, et un polymère éthylène-maléique anhydride chargé en molécules lourdes, formant une gelée, ayant un poids moléculaire d'au plus 1.000.000; ledit chromophore étant présent à une quantité d'environ 1.5 à 4.0 pour cent par poids, ledit polymère étant présent à une quantité d'environ 2 à 8 pour cent par poids, et ladite gelée a une viscosité d'environ 40.000 à 300.000 cps.

4). Un dosage ophtalmique d'une gelée acqueuse pour établir un blocage total de la transmission des UV. lorsqu'elle est placée dans les yeux, ladite gelée comprenant un mélange 50/50 sur une base de poids d'octyl méthoxycinnamate/4-t-butyl-4-méthoxy-dibenzoylméthane, et un polymère éthylene-maléique anhydride chargé en molécules lourdes formant une gelée, ayant un poids moléculaire d'au plus 1.000.000; ledit mélange chromophore étant présent à une quantité d'environ 1 à 8 pour cent par poids, ledit polymère étant présent à une quantité d'environ 2 à 8 pour cent par poids, et ladite gelée a une viscosité d'environ 40.000 à 300.000 cps.

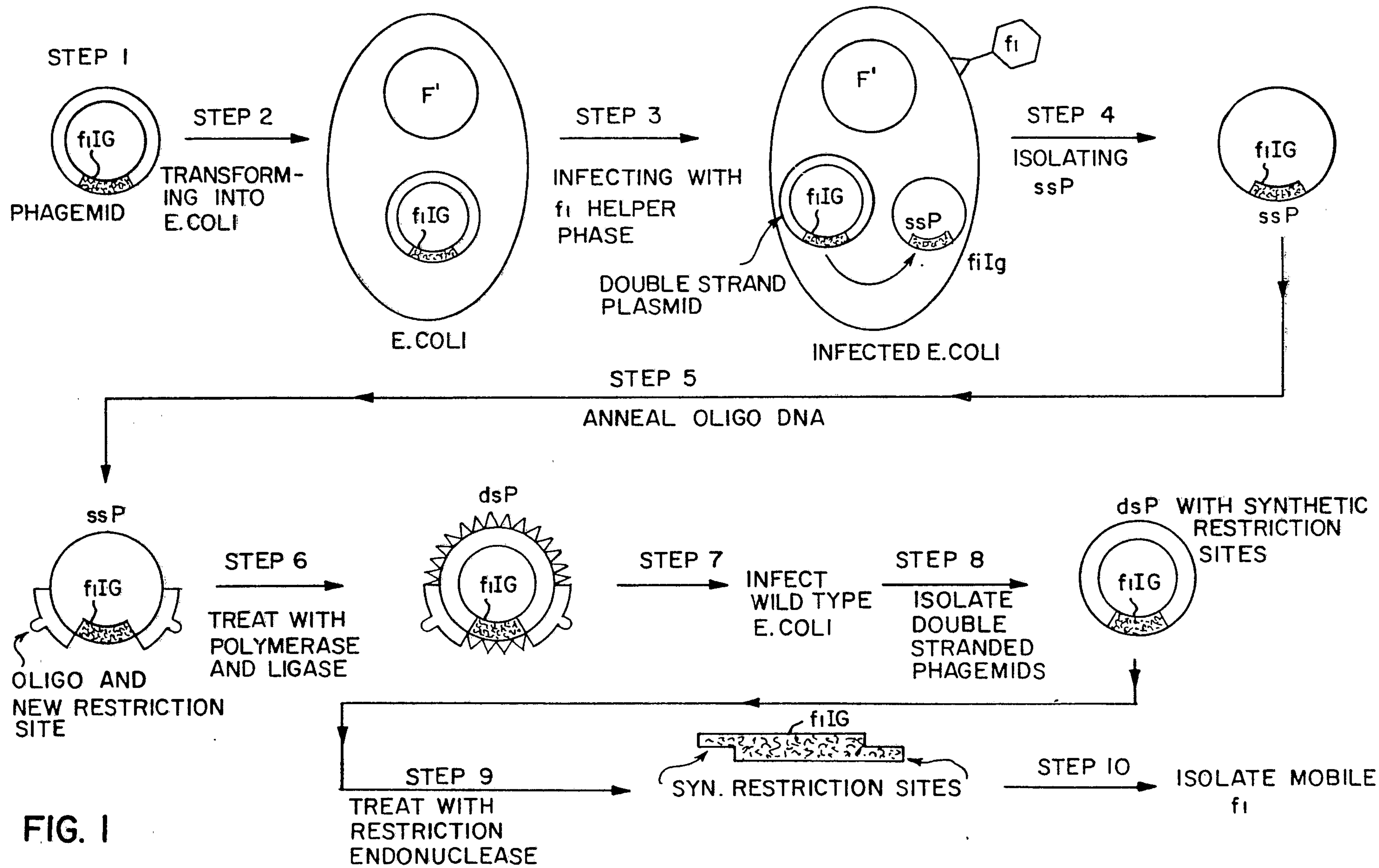
STEP I
PHAGEMID
f1IG
STEP 2
TRANSFORM-
ING INTO
E.COLI
F'
f1IG
E.COLI
STEP 3
INFECTING WITH
f1 HELPER
PHASE
DOUBLE STRAND
PLASMID
F'
f1
f1IG
ssP
f1Ig
INFECTED E.COLI
STEP 4
ISOLATING
ssP
f1IG
ssP
STEP 5
ANNEAL OLIGO DNA
ssP
f1IG
OLIGO AND
NEW RESTRICTION
SITE
STEP 6
TREAT WITH
POLYMERASE
AND LIGASE
dsP
f1IG
STEP 7
INFECT
WILD TYPE
E.COLI
STEP 8
ISOLATE
DOUBLE
STRANDED
PHAGEMIDS
dsP WITH SYNTHETIC
RESTRICTION
SITES
f1IG
STEP 9
TREAT WITH
RESTRICTION
ENDONUCLEASE
f1IG
SYN. RESTRICTION SITES
STEP 10
ISOLATE MOBILE
f1

FIG. I

Office européen des brevets

# RAPPORT DE RECHERCHE EUROPEENNE

Numero de la demande

EP 89 42 0308

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,X | US-A-4 765 977 (N.A. BARON)<br>* En entier *<br>--- | 1 | A 61 K 9/06<br>A 61 K 47/32<br>A 61 F 9/00 |
| A | FR-A- 707 525 (A.-M. HALPHEN)<br>* En entier *<br>--- | 1-4 | |
| A | US-A-4 407 792 (R.D. SCHOENWALD)<br>* Colonne 2, lignes 4-63; exemple 3 *<br>--- | 1-4 | |
| A | US-A-3 821 363 (A.S. BLACK)<br>* Colonne 1, ligne 65 - colonne 3, ligne 39; exemples 4,23 *<br>--- | 1-4 | |
| A | US-A-4 304 895 (S. LOSHAEK)<br>* Colonne 1, lignes 56-64; colonne 3, lignes 16-62 *<br>--- | 2 | |
| A | FR-A-2 440 933 (L. GIVAUDAN & CIE.)<br>* Colonne 1 - colonne 3, ligne 5; colonne 3, lignes 12-16; colonne 4, lignes 4-12,20-28; revendications 3-5 *<br>----- | 3,4 | |
| | | | **DOMAINES TECHNIQUES RECHERCHES (Int. Cl.5)** |
| | | | A 61 K |

Le présent rapport a été établi pour toutes les revendications

| Lieu de la recherche | Date d'achèvement de la recherche | Examinateur |
|---|---|---|
| LA HAYE | 30-11-1989 | MUELLNERS W. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)